# EUROPÄISCHE PATENTSCHRIFT

(11) **EP 1 658 088 B1**
(45) Veröffentlichungstag und Bekanntmachung des Hinweises auf die Patenterteilung: **27.03.2013**
(21) Anmeldenummer: 04740953.7
(22) Anmeldetag: 13.07.2004
(51) Int. Cl.: A61K 35/74, A61K 36/185, A61K 36/25, A61K 36/15, A61K 36/53, A61K 36/77, A61P 17/00, A61K 8/97, A61K 38/01, A61Q 19/00, A61Q 5/00

(54) **PRÄBIOTISCH WIRKSAME ZUSAMMENSETZUNG ENTHALTEND EXTRAKTE AUS PINUS SYLVESTRIS UND RIBES NIGRUM ZUR HEMMUNG DES WACHSTUMS VON PROPIONIBACTERIUM ACNES**
PREBIOTICALLY ACTIVE COMPOSITION COMPRISING EXTRACTS OF PINUS SYLVESTRIS AND RIBES NIGRUM FOR USE IN PREVENTING GROWTH OF PROPIONIBACTERIUM ACNES
COMPOSITION AVEC UN EFFECT PREBIOTIQUE COMPRENANT DES EXTRAITS DE PINUS SYLVESTRIS ET DE RIBES NIGRUM POUR L'INHIBITION DE LA CROISSANCE DE PRIOPIONIBACTERIUM ACNES

(30) Priorität: 21.07.2003 DE 10333245; 10.03.2004 DE 102004011968
(43) Veröffentlichungstag der Anmeldung: 24.05.2006
(73) Patentinhaber: Henkel AG & Co. KGaA, 40589 Düsseldorf (DE)
(72) Erfinder: BOCKMÜHL, Dirk, 42113 Wuppertal (DE); HÖHNE, Heide-Marie, 50189 Elsdorf (DE); JASSOY, Claudia, 40237 Düsseldorf (DE); SCHOLTYSSEK, Regine, 40882 Mettmann (DE); WALDMANN-LAUE, Marianne, 40789 Monheim (DE); SCHOLZ, Wolfhard, 47829 Krefeld (DE); SÄTTLER, Andrea, 40225 Düsseldorf (DE)
(86) Internationale Anmeldenummer: PCT/EP2004/007708
(87) Internationale Veröffentlichungsnummer: WO 2005/011716

(56) Entgegenhaltungen:
- EP-A- 1 050 300
- FR-A- 2 713 086
- US-A- 6 083 932
- DATABASE WPI Section Ch, Week 199914 Derwent Publications Ltd., London, GB; Class B04, AN 1999-165082 XP002306946 & KR 9 604 897 B1 (CHEIL FOODS & CHEM CO LTD) 17. April 1996 (1996-04-17)
- DATABASE WPI Section Ch, Week 199515 Derwent Publications Ltd., London, GB; Class B04, AN 1995-107491 XP002306947 & CN 1 079 646 A (DONGHU MATERIAL COMPOUND NEW TECHNOLOGY) 22. Dezember 1993 (1993-12-22)
- PATENT ABSTRACTS OF JAPAN Bd. 1995, Nr. 05, 30. Juni 1995 (1995-06-30) & JP 7 033673 A (TAISHO PHARMACEUT CO LTD), 3. Februar 1995 (1995-02-03)
- PATENT ABSTRACTS OF JAPAN Bd. 2003, Nr. 11, 5. November 2003 (2003-11-05) & JP 2003 201229 A (SHISEIDO CO LTD), 18. Juli 2003 (2003-07-18)
- DATABASE WPI Section Ch, Week 200368 Derwent Publications Ltd., London, GB; Class B04, AN 2003-718480 XP002306948 & KR 2002 093 251 A (ROSEE COSMETICS CO LTD) 16. Dezember 2002 (2002-12-16)
- PATENT ABSTRACTS OF JAPAN Bd. 2002, Nr. 02, 2. April 2002 (2002-04-02) & JP 2001 278775 A (KOSE CORP), 10. Oktober 2001 (2001-10-10)
- PATENT ABSTRACTS OF JAPAN Bd. 1995, Nr. 04, 31. Mai 1995 (1995-05-31) & JP 7 017846 A (SANSHO SEIYAKU CO LTD), 20. Januar 1995 (1995-01-20)
- DATABASE BIOSIS [Online] BIOSCIENCES INFORMATION SERVICE, PHILADELPHIA, PA, US; September 2001 (2001-09), HU S ET AL: "Effect of subcutaneous injection of ginseng on cows with subclinical Staphylococcus aureus mastitis" XP002306945 Database accession no. PREV200100513297 & JOURNAL OF VETERINARY MEDICINE SERIES B, Bd. 48, Nr. 7, September 2001 (2001-09), Seiten 519-528, ISSN: 0931-1793

## Beschreibung

Gegenstand der vorliegenden Erfindung ist eine kosmetische oder pharmazeutische Zusammensetzung, enthaltend eine Extraktmischung aus *Ribes nigrum* und *Pinus sylvestris,* sowie die Verwendung dieser Zusammensetzung.

Entzündliche Zustände der Haut werden durch schädliche Bakterien, beispielsweise *Propionibacterium acnes,* hervorgerufen, die permanent auf der Haut zu finden sind, sich aber unter bestimmten Bedingungen stärker vermehren und so beispielsweise zu "unreiner Haut" oder Akne führen. Es handelt sich hierbei also im wesentlichen um Mikroorganismen (Bakterien und Pilze), die als pathogen einzuordnen sind.
Die residente bakterielle Hautflora setzt sich dabei aber auch noch aus anderen Bakterienspezies zusammen, die nicht nur keinen Schaden anrichten, sondern vielmehr durch ihr Wachstum die Schadkeime in Schach halten und somit eine wichtige Schutzfunktion ausüben. Hierzu gehören vor allem Koagulase-negative Staphylokokken wie *S*. *epidermidis.* Im allgemeinen wird für die gutartigen, erwünschten Hautkeime auch der Begriff "saprophyt" gebraucht. Allerdings gibt es auch erwünschte Hautkeime, die nach der momentanen Klassifizierung nicht der Gruppe der "saprophyten" Hautkeime zugeordnet werden, ebenso wie es als "pathogen" eingestufte Keime gibt, die unter Umständen wünschenswert sind, so dass eine Abgrenzung zwischen "erwünscht" und "unerwünscht" nicht deckungsgleich ist mit einer Abgrenzung zwischen "saprophyt" und "pathogen". Es ist hierbei insbesondere etwa auch zu berücksichtigen, dass gutartige hautfreundliche Keime bei zu starker Vermehrung pathogen werden können. Die Einstufung der Bakterien als pathogen ist also gegebenenfalls von der Zusammensetzung der mikrobiellen Hautflora abhängig.

Unselektiv antibakterielle Wirkstoffe, wie sie beispielsweise zur Akneprävention und -bekämpfung in handelsüblichen Kosmetika eingesetzt werden, töten nicht nur die unerwünschten Hautkeime sondern auch erwünschte Hautkeime ab und führen so zu einer Störung des biologischen Gleichgewichts, was verschiedene unerwünschte Folgen haben kann.

Es besteht daher ein Bedarf, Mittel zur Verfügung zu haben, die selektiv am Applikationsort Haut das Wachstum und/oder die Überlebensfähigkeit der wünschenswerten Keime der Hautflora gegenüber dem Wachstum und/oder der Überlebensfähigkeit der unerwünschten Keime der Hautflora fördern. Solche Substanzen werden auch als "präbiotisch" bezeichnet.

Der Nachweis eines präbiotischen Effektes von Substanzen ist bislang im wesentlichen beschränkt auf das Intestum. So ist in verschiedenen Publikationen die Verwendung von Substanzen, die förderlich auf das Wachstum wünschenswerter Darmbakterien sind, beschrieben. Ahn et al. (1990) Microbial Ecology in Health and Disease 3, 223-229, beschreibt hierbei insbesondere auch die Verwendung eines Ginseng-Extraktes.

Für die Haut wurde bislang lediglich berichtet, dass ein Oligosaccharid einen präbiotischen Effekt bewirkt (Werbebroschüre über BioEcolia® der Solabia Group, Frankreich), indem es selektiv von saprophyten Bakterien bevorzugt verwertet werden kann. Es wurde hierbei gezeigt, dass das verwendete Oligosaccharid das Wachstum von *Micrococcus kristinae* sowohl gegenüber dem Wachstum von *Staphylococcus aureus* als auch gegenüber dem Wachstum von *Corynebakterium xerosis* fördert.

Desweiteren wurde in EP1050300 berichtet, dass eine Mischung aus Farnesol und Xylitol als präbiotische Substanz verwendet werden kann, da diese Mischung selektiv antibakteriell gegenüber *S*. *aureus* wirkt und sich so der konkurrierende Keim *S*. *epidermidis* besser vermehren kann.

Aufgabe der vorliegenden Erfindung war es nun, weitere Substanzen ausfindig zu machen, die auf der Haut bzw. in der Hautflora präbiotisch wirksam sind.

Überraschenderweise wurden nun Pflanzenextrakte gefunden, die auf der Haut einen präbiotischen Effekt bewirken und daher vorteilhaft zur Hautbehandlung eingesetzt werden können.

Unter präbiotischem Effekt ist erfindungsgemäß zu verstehen, dass das Wachstum und/oder die Überlebensfähigkeit der erwünschten, insbesondere hautfreundlichen, Hautkeime bzw. Mikroflora gegenüber dem Wachstum und/oder der Überlebensfähigkeit der unerwünschten, insbesondere hautfeindlichen, Hautkeime bzw. Mikroflora gefördert wird. Dies kann sowohl dadurch erreicht werden, dass der Wirkstoff förderlich auf das Wachstum der erwünschten Hautkeime wirkt, ohne unmittelbar Einfluss auf das Wachstum der unerwünschten Hautkeime zu nehmen, als auch dadurch, dass der Wirkstoff hemmend auf das Wachstum der unerwünschten Hautkeime ist, ohne unmittelbar Einfluss auf das Wachstum der erwünschten Hautkeime zu nehmen. In einer erfindungsgemäß besonders bevorzugten und besonders überraschenden Ausführungsform jedoch wirkt der Wirkstoff förderlich auf das Wachstum der erwünschten Hautkeime und wirkt zugleich hemmend auf das Wachstum der unerwünschten Hautkeime.

Erfindungsgemäß sind unter dem Begriff "Haut" bevorzugt die Haut selbst, insbesondere die menschliche Haut, daneben aber auch die Schleimhaut sowie Hautanhangsgebilde, sofern sie lebende Zellen umfassen, insbesondere Haarfollikel, Haarwurzel, Haarzwiebel, das ventrale Epithel des Nagelbetts (Lectulus) sowie Talgdrüsen und Schweißdrüsen zu verstehen.

Gegenstand der vorliegenden Erfindung ist die Verwendung eines Extrakts aus *Ribes nigrum* zur Herstellung topischer pharmazeutischer oder kosmetischer Zusammensetzungen zur Förderung des Wachstums Koagulase-negativer Staphylokokken, ausgewählt aus *S*. *epidermidis* und *S*. *wameri,* und zur gleichzeitigen Hemmung des Wachstums von *Propionibacterium acnes* auf der Haut.
Vorzugsweise ist das erfindungsgemäße, auf der Haut präbiotisch wirksame Pflanzenextrakt dazu geeignet, das natürlicherweise vorkommende gesunde mikrobielle Gleichgewicht der Hautflora wiederherzustellen bzw. zu stabilisieren. Gegenstand der vorliegenden Erfindung ist desweiteren die Verwendung eines Extrakts aus *Ribes nigrum* zur Herstellung topischer pharmazeutischer oder kosmetischer Zusammensetzungen zur Behandlung von unreiner oder fettiger Haut, insbesondere zur Behandlung von Akne. Die Behandlung kann hierbei, wie auch bei anderen Anwendungsgebieten, präventiv erfolgen.
Gegenstand der vorliegenden Erfindung ist des weiteren eine kosmetische oder pharmazeutische Zusammensetzung, enthaltend eine Extraktmischung aus *Ribes nigrum* und *Pinus sylvestris,* wobei es sich bei der kosmetischen oder pharmazeutischen Zusammensetzung vorzugsweise um ein topisches Hautbehandlungsmittel handelt.
Die Extraktmischung aus *Ribes nigrum* und *Pinus sylvestris* ist hierbei vorzugsweise in einer Menge von 0,01 bis 20, besonders bevorzugt von 0,05 bis 10, vor allem von 0,1 bis 5, insbesondere von 0,5 bis 2 Gew.-%, bezogen auf das Gesamtgewicht der Zusammensetzung, in der Zusammensetzung enthalten.
Besonders bevorzugt handelt es sich bei dem Pflanzenextrakt um ein Extrakt aus *Picea spp.,* insbesondere um ein Extrakt aus *Picea excelsa* (synonyom *Picea abies,* Fichte) oder aus *Picea glauca* (Zuckerhutfichte, Norway Spruce), aus *Pinus* sp., insbesondere aus *Pinus sylvestris,* aus *Paullinia sp.* (Guarana), insbesondere *Paullinia cubana, aus Panax sp.,* insbesondere *Panax ginseng* (Ginseng), aus *Lamium sp.,* insbesondere *Lamium album* (White Nettle), oder aus *Ribes sp.,* insbesondere *Ribes nigrum* (Blackcurrant, Schwarze Johannisbeere), oder es handelt sich um Mischungen davon.
Die Herstellung des präbiotisch wirksamen Pflanzenextraktes kann in jeder dem Fachmann bekannten Weise unter Verwendung jedes beliebigen Pflanzengewebes und unter Verwendung jedes beliebigen Extraktionsmittels erfolgen. So kann der Pflanzenextrakt beispielsweise durch Extraktion der gesamten Pflanze, durch Exktraktion aus Blüten, Blättern, Samen, Wurzeln und/oder durch Extraktion aus dem Meristem der Pflanze erfolgen.

Als Extraktionsmittel zur Herstellung der genannten Pflanzenextrakte können beispielsweise Wasser, Alkohole sowie deren Mischungen verwendet werden. Als Alkohole kommen beispielsweise niedere Alkohole wie Ethanol und Isopropanol, insbesondere aber auch mehrwertige Alkohole wie Ethylenglykol, Propylenglykol und Butylenglykol in Frage, und zwar sowohl als alleiniges Extraktionsmittel als auch in Mischung mit Wasser. So haben sich beispielsweise Pflanzenextrakte auf Basis von Wasser/Propylenglykol im Verhältnis 1:10 bis 10:1 als besonders geeignet erwiesen. Die Extraktion kann beispielsweise in Form von Wasserdampfdestillation durchgeführt werden. Gegebenenfalls kann auch eine Trockenextraktion erfolgen.

Bei dem Extrakt aus Nadelbäumen, und insbesondere aus Pinaceae, handelt es sich erfindungsgemäß bevorzugt um ein Extrakt aus den Nadeln oder aus der Rinde. Bei

Bei dem Extrakt aus Blackcurrant (*Ribes nigrum),* handelt es sich vorzugsweise um ein Wasser/Propylenglykol-Extrakt, vor allem aus den Blättern.

Die auf der Haut präbiotisch wirksamen Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/-/caprinsäureglyceride, Polyoxyethylenglycerinmonolaurat und Polyoxyethylenglykolkokosfettsäureglyceride.

Bei der erfindungsgemäßen kosmetischen oder pharmazeutischen Zusammensetzung kann es sich um jede beliebige Darreichungsform handeln, beispielsweise um eine Seife, eine Lotion, ein Spray, eine Creme, ein Gel, eine Emulsion, eine Reinigungsflüssigkeit oder Reinigungsmilch, ein Deodorant, ein Antitranspirant, eine Salbe, eine Haarkur oder ein Shampoo und sie kann auch in jeder der beschriebenen oder sonstigen Darreichungsformen enthalten sein, beispielsweise auch in einem Pflaster, insbesondere in einem Gel-Reservoir- oder Matrixpflaster.

Als Applikationsort kommt die Haut jedes Körperbereichs in Frage, insbesondere die Gesichtshaut, die Kopfhaut, die Haut an den Füßen und Händen, die Haut in den Achselhöhlen sowie die vaginale Mucosa.

Die erfindungsgemäße kosmetische oder pharmazeutische Zusammensetzung kann auch weitere Bestandteile enthalten. In einer bevorzugten Ausführungsform enthält sie mindestens eine der im folgenden aufgezählten Substanzen. Sie kann auch jede beliebige Kombination der im folgenden aufgezählten Bestandteile enthalten.

In einer bevorzugten Ausführungsform enthält die erfindungsgemäße Zusammensetzung mindestens eine Substanz ausgewählt aus Vitaminen, Provitaminen oder Vitaminvorstufen der Vitamin B-Gruppe oder deren Derivaten sowie den Derivaten von 2-Furanon.

Zur Vitamin B-Gruppe oder zu dem Vitamin B-Komplex gehören unter anderem
• Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
• Vitamin B₂, Trivialname Riboflavin, chemische Bezeichung 7,8-Dimethyl-10-(1-D-ribityl)-benzo[g]pteridin-2,4(3*H*,10*H*)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt. Bevorzugt werden Riboflavin oder seine Derivate in Mengen von 0,05 bis 1 Gew.-%, bezogen auf das gesamte Mittel, eingesetzt.
• Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
• Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden. 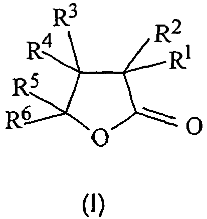 Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom; R³ und R⁴ für eine Methylgruppe und R⁵ und R⁶ für ein Wasserstoffatom stehen. Das Stereoisomer (R)-Pantolacton entsteht beim Abbau von Pantothensäure.
   Die genannten Verbindungen des Vitamin B₅-Typs sowie die 2-Furanonderivate sind in den erfindungsgemäßen Mitteln bevorzugt in einer Gesamtmenge von 0,05 bis 10 Gew.-%, bezogen auf das gesamte Mittel, enthalten. Gesamtmengen von 0,1 bis 5 Gew.-% sind besonders bevorzugt.
• Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
• Vitamin B₇ (Biotin), auch als Vitamin H oder "Hautvitamin" bezeichnet. Bei Biotin handelt es sich um (3aS,4S, 6aR)-2-Oxohexahydrothienol[3,4-d]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Panthenol, Pantolacton, Nicotinsäureamid sowie Biotin sind erfindungsgemäß ganz besonders bevorzugt.

Ein weiterer Gegenstand der Erfindung ist ein kosmetisches oder pharmazeutisches Hautbehandlungsmittel, enthaltend ein präbiotisches Pflanzenextrakt und weiterhin mindestens ein weiteres Pflanzenextrakf. Dieses weitere Pflanzenextrakt kann beispielsweise durch Extraktion der gesamten Pflanze, aber auch ausschließlich durch Exktraktion aus Blüten und/oder Blättern und/oder Samen und/oder anderen Pflanzenteilen, hergestellt werden. Erfindungsgemäß sind vor allem die Extrakte aus dem Meristem, also dem teilungsfähigen Bildungsgewebe der Pflanzen, und die Extrakte aus speziellen Pflanzen wie Grünem Tee, Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Huflattich, Fünffingerkraut, Ingwerwurzel und Süßkartoffel als weiteres Pflanzenextrakt bevorzugt. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.

Als weiteres Pflanzenextrakt besonders bevorzugt sind die Extrakte aus Spirulina, Grünem Tee, Aloe Vera, Meristem, Hamamelis, Aprikose, Ringelblume, Guave, Süßkartoffel, Limette, Mango, Kiwi, Gurke, Malve, Eibisch und Veilchen. Die erfindungsgemäßen Mittel können als zusätzliches Pflanzenextrakt auch Mischungen aus mehreren, insbesondere aus zwei, verschiedenen Pflanzenextrakten enthalten.

Als Extraktionsmittel zur Herstellung der genannten weiteren Pflanzenextrakte können ebenso wie zur Herstellung der präbiotisch wirksamen Pflanzenextrakte beispielsweise Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Wasserdampfdestillation fällt erfindungsgemäß unter die bevorzugten Extraktionsverfahren. Die Extraktion kann aber gegebenenfalls auch in Form von Trockenextraktion erfolgen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/-/caprinsäureglyceride, Polyoxyethylenglycerinmonolaurat und Polyoxyethylenglykolkokosfettsäureglyceride.

Weiterhin kann es bevorzugt sein, in den erfindungsgemäßen Mitteln Mischungen aus mehreren, insbesondere aus zwei, verschiedenen Pflanzenextrakten zusätzlich zu dem präbiotisch wirksamen Pflanzenextrakt einzusetzen.

Hinsichtlich der erfindungsgemäß verwendbaren Pflanzenextrakte wird weiterhin auf die Extrakte hingewiesen, die in der auf Seite 44 der 3. Auflage des Leitfadens zur Inhaltsstoffdeklaration kosmetischer Mittel, herausgegeben vom lndustrieverband Körperpflege- und Waschmittel e.V. (IKW), Frankfurt, beginnenden Tabelle aufgeführt sind.

Ein weiterer Gegenstand der Erfindung ist ein kosmetisches oder pharmazeutisches Hautbehandlungsmittel, enthaltend ein präbiotisches Pflanzenextrakt und mindestens eine MMP-1-inhibierende Substanz ausgewählt aus Photolyase und/oder T4 Endonuclease V, Propylgallat, Precocenen, 6-Hydroxy-7-methoxy-2,2-dimethyl-1(2H)-benzopyran, 3,4-Dihydro-6-hydroxy-7-methoxy-2,2-dimethyl-1(2H)-benzopyran (als Handelsprodukt Lipochroman 6™ von der Firma Lipotec SA erhältlich) und deren Gemischen. Precocene sind in Pflanzen vorkommende Chromen-Derivate, die als Hormone bekannt sind (The Merck Index, 12. Auflage, Merck & Co. 1996). Die MMP-1-inhibierende Wirkung dieser Substanzen ist in der deutschen Offenlegungsschrift DE 10016016 A1 beschrieben. Sie werden in Mengen von 0,1 bis 5, vorzugsweise von 0,5 bis 2 Gew.-%, jeweils bezogen auf das gesamte Mittel, eingesetzt.

In einer besonders bevorzugten Ausführungsform enthalten die erfindungsgemäßen Hautbehandlungsmittel zusätzlich mindestens einen Ester von Retinol (Vitamin A₁) mit einer C₂₋₁₈-Carbonsäure. Bevorzugte Retinolester sind Retinylacetat und Retinylpalmitat, besonders bevorzugt ist Retinylpalmitat. Die Retinolester werden in Mengen von 0,1 bis 5, vorzugsweise von 0,5 bis 2 Gew.-%, jeweils bezogen auf das gesamte Mittel, eingesetzt.

In einer weiteren bevorzugten Ausführungsform, insbesondere bei der Verwendung als Emulsion oder tensidische Lösung, vor allem als Reinigungsmittel, enthalten die erfindungsgemäßen Hautbehandlungsmittel mindestens eine oberflächenaktive Substanz als Emulgator oder Dispergiermittel. Emulgatoren bewirken an der Phasengrenzfläche die Ausbildung von wasser- bzw. ölstabilen Adsorptionsschichten, die die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. W/O-Emulsionen, die ohne hydrophile Emulgatoren stabilisiert sind, sind in den Offenlegungsschriften DE 19816665 A1 und DE 19801593 A1 offenbart. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Erfindungsgemäß verwendbare Emulgatoren sind beispielsweise
- Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole,
- C₁₂-C₂₂-Fettsäuremono- und -diester von Anlagerungsprodukten von 1 bis 30 Mol Ethylenoxid an C₃-C₆-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten C₈-C₂₂-Fettsäuren,
- Sterole (Sterine). Als Sterole wird eine Gruppe von Steroiden verstanden, die am C-Atom 3 des Steroid-Gerüstes eine Hydroxylgruppe tragen und sowohl aus tierischem Gewebe (Zoosterole) wie auch aus pflanzlichen Fetten (Phytosterole) isoliert werden. Beispiele für Zoosterole sind das Cholesterol und das Lanosterol. Beispiele geeigneter Phytosterole sind Beta-Sitosterol, Stigmasterol, Campesterol und Ergosterol. Auch aus Pilzen und Hefen werden Sterole, die sogenannten Mykosterole, isoliert.
- Phospholipide, vor allem die Glucose-Phospolipide, die z. B. als Lecithine bzw. Phosphatidylcholine aus z. B. Eidotter oder Pflanzensamen (z. B. Sojabohnen) gewonnen werden,
- Fettsäureester von Zuckern und Zuckeralkoholen wie Sorbit,
- Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI),
- Lineare und verzweigte C₈-C₃₀-Fettsäuren und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugten Ausführungsform ist mindestens ein nichtionischer Emulgator mit einem HLB-Wert von 8 und darunter, gemäß den im Römpp-Lexikon Chemie (Eds.: J. Falbe, M. Regitz), 10. Auflage, Georg Thieme Verlag Stuttgart, New York, (1997), Seite 1764, aufgeführten Definitionen des HLB-Wertes, enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Als Emulgator der Formel R¹ - O - R² besonders bevorzugt ist ein Behen- oder Erucylderivat, in welchem R¹ eine lineare, endständig substituierte Alkyl-, Alkenyl- oder Acylgruppe mit 22 C-Atomen darstellt.

Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitan-monobehenat oder Pentaerythrit-monoerucat.

In einer anderen, ebenfalls besonders bevorzugten Ausführungsform ist mindestens ein ionischer Emulgator, ausgewählt aus anionischen, zwitterionischen, ampholytischen und kationischen Emulgatoren, enthalten. Bevorzugte anionische Emulgatoren sind Alkylsulfate, Alkylpolyglycolethersulfate und Ethercarbonsäuren mit 10 bis 18 C-Atomen in der Alkylgruppe und bis zu 12 Glycolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate. Zwitterionische Emulgatoren tragen im Molekül mindestens eine quartäre Ammoniumgruppe und mindestens eine -COO⁻ - oder -SO₃⁻ - Gruppe. Besonders geeignete zwitterionische Emulgatoren sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Ampholytische Emulgatoren enthalten außer einer C₈- C₂₄-Alkyl- oder -Acylgruppe mindestens eine freie Aminogruppe und mindestens eine -COOH- oder -SO₃H-Gruppe im Molekül und können innere Salze ausbilden. Beispiele für geeignete ampholytische Emulgatoren sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Die ionischen Emulgatoren sind in einer Menge von 0,01 bis 5 Gew.-%, bevorzugt von 0,05 bis 3 Gew.-% und besonders bevorzugt von 0,1 bis 1 Gew.-%, bezogen auf das gesamte Mittel, enthalten.

Weiterhin können die erfindungsgemäßen Zusammensetzungen schäumende nichtionische, zwitterionische, anionische und kationische Tenside enthalten. Beispiele für nichtionische Tenside sind
- alkoxylierte Fettsäurealkylester der Formel R¹CO-(OCH₂CHR²)ₓOR³,
   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈ - C₂₂-Alkylamin-N-oxide,
- Alkylpolygykoside entsprechend der allgemeinen Formel RO-(Z)ₓ wobei R für eine C₈- C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.
   Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether-und Amidgruppen sowie Hydroxylgruppen enthalten sein. Beispiele für geeignete schäumende Aniontenside sind, jeweils in Form der Natrium-, Kalium- und Ammonium- sowie der Mono-, Di- und Trialkanolammoniumsalze mit 2 bis 4 Kohlenstoffatomen in der Alkanolgruppe,
- Acylglutamate der Formel (II), 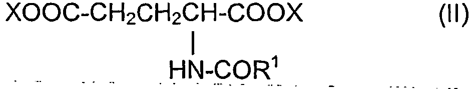 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (III), 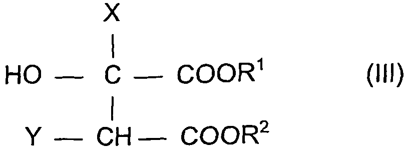 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten(C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, daß wenigstens eine der Gruppen R, R¹ oder R² ein Rest Z ist,
- Ester des Sulfobernsteinsäure-Salzes der allgemeinen Formel (IV), 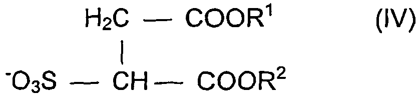 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen(C₆-C₁₆)-Hydroxyalkylpolyole mit 2 bis 16 Hydroxylresten ausgewählt ist, unter der Maßgabe, daß wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Ethoxygruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcosinate mit einem linearen oder verzweigten Acylrest mit 6 bis 22 Kohlenstoffatomen und 0, 1, 2 oder 3 Doppelbindungen,
- Acyltaurate mit einem linearen oder verzweigten Acylrest mit 6 bis 22 Kohlenstoffatomen und 0, 1, 2 oder 3 Doppelbindungen,
- Acylisethionate mit einem linearen oder verzweigten Acylrest mit 6 bis 22 Kohlenstoffatomen und 0, 1, 2 oder 3 Doppelbindungen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)_{z}-SO₃X, in der R eine bevorzugt lineare Alkylgruppe mit 8 bis 30 C-Atomen, besonders bevorzugt mit 8 - 18 C-Atomen, z = 0 oder 1 bis 12, besonders bevorzugt 3, und X ein Natrium-, Kalium-, Magnesium-, Zink-, Ammoniumion oder ein Monoalkanol-, Dialkanol- oder Trialkanolammoniumion mit 2 bis 4 Kohlenstoffatomen in der Alkanolgruppe ist, wobei ein besonders bevorzugtes Beispiel Zinkcocoylethersulfat mit einem Ethoxylierungsgrad von z = 3 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Alkyl- und/oder Alkenyletherphosphate der Formel (V) , 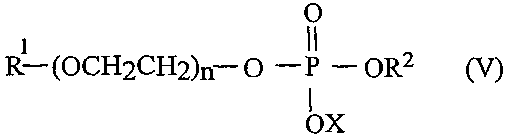
- in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht,
- sulfatierte Fettsäurealkylenglykolester der Formel R⁷CO(AlkO)ₙSO₃M, in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (VI), 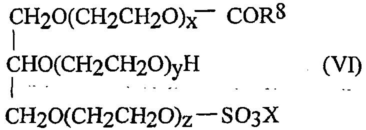
- in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VI) eingesetzt, in der R⁸CO für einen linearen Acylrest mit 8 bis 18 Kohlenstoffatomen steht.

In einer weiteren bevorzugten Ausführungsform enthalten die erfindungsgemäßen kosmetischen Zusammensetzungen mindestens einen organischen oder mineralischen oder modifizierten mineralischen Lichtschutzfilter. Bei den Lichtschutzfiltern handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.

Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlösliche sein. Erfindungsgemäß besonders bevorzugte öllösliche UV-Filtersind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb^{□} HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethylcyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Weiterhin hat es sich als besonders vorteilhaft erwiesen, dass die erfindungsgemäßen Hautbehandlungsmittel mindestens ein Proteinhydrolysat oder dessen Derivat enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda).

An Stelle der Proteinhydrolysate können zum einen anderweitig erhaltene Aminosäuregemische, zum anderen auch einzelne Aminosäuren sowie deren physiologisch verträgliche Salze eingesetzt werden. Zu den erfindungsgemäß bevorzugten Aminosäuren gehören Glycin, Serin, Threonin, Cystein, Asparagin, Glutamin, Pyroglutaminsäure, Alanin, Valin, Leucin, Isoleucin, Prolin, Tryptophan, Phenylalanin, Methionin, Asparaginsäure, Glutaminsäure, Lysin, Arginin und Histidin sowie die Zinksalze und die Säureadditionssalze der genannten Aminosäuren.
Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß einsetzbar sind auch kationisierte Proteinhydrolysate, wobei das zugrunde liegende Proteinhydrolysat vom Tier, von der Pflanze, von marinen Lebensformen oder von biotechnologisch gewonnenen Proteinhydrolysaten, stammen kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate seien einige der unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen, Steardimonium Hydroxypropyl Hydrolyzed Hair Keratin, Lauryldimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed- Wheat Protein, Cocodimonium Hydroxypropyl Silk Amino Acids, Hydroxypropyl Arginine Lauryl/Myristyl Ether HCl, Hydroxypropyltrimonium Gelatin. Ganz besonders bevorzugt sind die kationischen Proteinhydrolysate und -derivate auf pflanzlicher Basis.

In den erfindungsgemäßen Mitteln sind die Proteinhydrolysate und deren Derivate beziehungsweise die Aminosäuren und deren Derivate in Mengen von 0,01 - 10 Gew.-%, bezogen auf das gesamte Mittel enthalten. Mengen von 0,1 bis 5 Gew.%, insbesondere 0,1 bis 3 Gew.-%, sind besonders bevorzugt.

Weiterhin hat es sich als vorteilhaft erwiesen, dass die erfindungsgemäßen Hautbehandlungsmittel mindestens ein Mono-, Oligo- oder Polysaccharid oder deren Derivate enthalten.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B: Dextransulfat. Ebenfalls bevorzugt sind nichtionische Cellulose-Derivate, wie Methylcellulose, Hydroxypropylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide aus Fucose-Einheiten, z. B. das Handelsprodukt Fucogel^{®}. Besonders bevorzugt sind die aus Aminozuckereinheiten aufgebauten Polysaccharide, insbesondere Chitine und ihre deacetylierten Derivate, die Chitosane, und Mucopolysaccharide. Zu den erfindungsgemäß bevorzugten Mucopolysacchariden gehören Hyaluronsäure und ihre Derivate, z. B. Natriumhyaluronat oder Dimethylsilanolhyaluronat, sowie Chondroitin und seine Derivate, z. B. Chondroitinsulfat.

In einer besonders vorteilhaften Ausführungsform enthalten die erfindungsgemäßen Hautbehandlungsmittel mindestens ein filmbildendes, emulsionsstabilisierendes, verdickendes oder adhäsives Polymer, ausgewählt aus natürlichen und synthetischen Polymeren, die kationisch, anionisch, amphoter geladen oder nichtionisch sein können.

Erfindungsgemäß bevorzugt sind kationische, anionische sowie nichtionische Polymere.

Unter den kationischen Polymeren bevorzugt sind Polysiloxane mit quaternären Gruppen, z. B. die Handelsprodukte Q2-7224 (Dow Corning), Dow Corning^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere besonders bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

In einer weiteren bevorzugten Ausführungsform der Erfindung kann die Wirkung der erfindungsgemäßen Mittel durch Fettstoffe weiter optimiert werden. Geeignete Fettstoffe sind zum Beispiel:
- pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls,
- flüssige Paraffinöle, lsoparaffinöle und synthetische Kohlenwasserstoffe, z. B. 1,3-Di-(2-ethyl-hexyl)-cyclohexan (Cetiol^{®} S) oder Polydecen,
- Di-n-alkylether mit insgesamt 12 bis 36, insbesondere 12 bis 24 C-Atomen, z. B. Di-n-octylether (Cetiol^{®} OE), Di-n- n-Hexyl-n-octylether und n-Octyl-n-decylether.
- Fettsäuren, besonders lineare und/oder verzweigte, gesättigte und/oder ungesättigte C₈₋₃₀-Fettsäuren. Bevorzugt sind C₁₀₋₂₂-Fettsäuren. Beispiele sind die lsostearinsäuren und lsopalmitinsäuren wie die unter der Handelsbezeichnung Edenor^{®} vertriebenen Fettsäuren. Weitere typische Beispiele für solche Fettsäuren sind Capronsäure, Caprylsäure, 2-Ethylhexansäure, Caprinsäure, Laurinsäure, lsotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt sind üblicherweise die Fettsäureschnitte, die aus Cocosöl oder Palmöl erhältlich sind; insbesondere bevorzugt ist der Einsatz von Stearinsäure.
- Fettalkohole, besonders gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z. B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, z. B. 2-Ethylhexanol, wobei diese Aufzählung beispielhaften und nicht limitierenden Charakter haben soll.
- Esteröle, das heißt, Ester von C₆₋₃₀-Fettsäuren mit C₂₋₃₀-Fettalkoholen. Bevorzugt sind die Monoester der Fettsäuren mit Alkoholen mit 2 bis 24 C-Atomen. Als Alkohol- und Säurekomponenten der Esteröle können die vorstehend genannten Substanzen verwendet werden. Erfindungsgemäß besonders bevorzugt sind Isopropylmyristat, lsononansäure-C₁₆₋₁₈-alkylester, 2-Ethylhexylpalmitat, Stearinsäure-2-ethylhexylester, Cetyloleat, Glycerintricaprylat, Kokosfettalkoholcaprinat/-caprylat, n-Butylstearat, Oleylerucat, Isopropylpalmitat, Oleyloleat, Laurinsäurehexylester, Di-n-butyladipat, Myristylmyristat, Cetearyl Isononanoate und Ölsäuredecylester.
- Hydroxycarbonsäurealkylester, wobei die Vollester der Glycolsäure, Milchsäure, Äpfelsäure, Weinsäure oder Citronensäure bevorzugt sind, aber auch Ester der ß-Hydroxypropionsäure, der Tartronsäure, der D-Gluconsäure, Zuckersäure, Schleimsäure oder Glucuronsäure geeignet sind und besonders bevorzugt die Ester von C₁₂-C₁₅-Fettalkoholen, z. B. die Handelsprodukte Cosmacol^{®} der EniChem, Augusta Industriale, sind,
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykoldi(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, z. B. Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, z. B. Monomuls^{®} 90-018, Monomuls^{®} 90-L12 oder Cutina^{®} MD,
- Wachse, insbesondere Insektenwachse wie Bienenwachs und Hummelwachs, Pflanzenwachse wie Candelillawachs und Carnaubawachs, Fruchtwachse, Ozokerit, Mikrowachs, Ceresin, Paraffin, Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, synthetische Vollester aus Fettsäuren und Glykolen (z. B. Syncrowachs^{®}) oder Polyolen mit 2 - 6 C-Atomen, Ester von gegebenenfalls hydroxylierten C₂₋₄-Carbonsäuren mit Lanolinalkoholen und C₁₂₋₁₈-Fettalkoholen, Cholesterol- oder Lanosterolester von C₁₀₋₃₀-Fettsäuren, ethoxylierte C₁₂₋₂₀-Fettsäureglykolester, Fettsäuremonoalkanolamide mit einem C₁₂₋₂₂-Acylrest und einem C₂₋₄-Alkanolrest, synthetische Fettsäure-Fettalkoholestern, z. B. Stearylstearat oder Cetylpalmitat sowie Esterwachse aus natürlichen Fettsäuren und synthetischen C₂₀₋₄₀-Fettalkoholen (INCI-Bezeichnung C20-40 Alkyl Stearate),
- Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane, bevorzugt die Substanzen mit der INCI-Bezeichnung Dimethicone Copolyol, sowie Polydialkylsiloxane, die Amin- und/oder Hydroxy-Gruppen enthalten.

Die Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf das gesamte Mittel.

In einer weiteren bevorzugten Ausführungsform enthält das Hautbehandlugsmittel mindestens eine α-Hydroxycarbonsäure oder α-Ketocarbonsäure oder deren Ester-, Lacton- oder Salzform. Geeignete α-Hydroxycarbonsäuren oder α-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

Die erfindungsgemäßen Mittel können weitere Wirk-, Hilfs- und Zusatzstoffe enthalten, beispielsweise:
- Vitamine, Provitamine und Vitaminvorstufen aus den Gruppen A, C, E und F, insbesondere 3,4-Didehydroretinol (Vitamin A₂), β-Carotin (Provitamin des Vitamin A₁), Ascorbinsäure (Vitamin C), sowie die Palmitinsäureester, Glucoside oder Phosphate der Ascorbinsäure, Tocopherole, insbesondere α-Tocopherol sowie seine Ester, z. B. das Acetat, das Nicotinat, das Phosphat und das Succinat; weiterhin Vitamin F, worunter essentielle Fettsäuren, besonders Linolsäure, Linolensäure und Arachidonsäure, verstanden werden;
- Allantoin,
- Bisabolol,
- Antioxidantien, zum Beispiel Imidazole (z. B. Urocaninsäure) und deren Derivate, Peptide wie D,L-Carnosin, D-Carnosin, L-Carnosin - und deren Derivate (z. B. Anserin), Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, das Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Katalase, Superoxid-Dismutase, Zink und dessen Derivate (z. B. ZnO, ZnSO₄), Selen und dessen Derivate (z. B. Selen-Methionin), Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe,
- Ceramide und Pseudoceramide,
- Triterpene, insbesondere Triterpensäuren wie Ursolsäure, Rosmarinsäure, Betulinsäure, Boswelliasäure und Bryonolsäure,
- Monomere Catechine, besonders Catechin und Epicatechin, Leukoanthocyanidine, Catechinpolymere (Catechin-Gerbstoffe) sowie Gallotannine,
- Verdickungsimittel, z. B. Gelatine, pflanzengumme wie Agar-Agar, Guar-Gum, Alginate, Xanthan-Gum, Gummi arabicum, Karaya-Gummi oder Johannisbrotkernmehl, natürliche und synthetische Tone und Schichtsilikate, z. B. Bentonit, Hectorit, Montmorillonit oder Laponite^{®}, vollsynthetische Hydrokolloide wie z. B. Polyvinylalkohol, und außerdem Ca-, Mg- oder Zn-Seifen von Fettsäuren,
- Pflanzenglycoside,
- Strukturanten wie Maleinsäure und Milchsäure,
- Dimethylisosorbid,
- Alpha-, beta- sowie gamma-Cyclodextrine, insbesondere zur Stabilisierung von Retinol,
- Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate
- Parfümöle, Pigmente sowie Farbstoffe zum Anfärben des Mittels,
- Substanzen zur Einstellung des pH-Wertes, z. B. α- und β-Hydroxycarbonsäuren,
- Komplexbildner wie EDTA, NTA, β-Alanindiessigsäure und Phosphonsäuren,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Treibmittel wie Propan-Butan-Gemische, N₂O, Dimethylether, CO₂ und Luft.

In einer bevorzugten Ausführungsform liegen die erfindungsgemäßen Hautbehandlungsmittel in Form einer flüssigen oder festen Öl-in-Wasser-Emulsion, Wasser-in-Öl-Emulsion, Mehrfach-Emulsion, Mikroemulsion, PIT-Emulsion oder Pickering-Emulsion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-ÖI(W/O)-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. Einzelheiten bezüglich dieser "PIT-Emulsionen" z. B. der Druckschrift Angew. Chem. 97, 655 - 669 (1985) zu entnehmen.

In der erfindungsgemäßen Ausführungsform als Antitranspirant können Antitranspirant-Wirkstoffe enthalten sein. Als Antitranspirant-Wirkstoffe eignen sich erfindungsgemäß wasserlösliche adstringierende oder einweißkoagulierende metallische Salze, insbesondere anorganische und organische Salze des Aluminiums, Zirkoniums, Zinks und Titans sowie beliebige Mischungen dieser Salze. Erfindungsgemäß wird unter Wasserlöslichkeit eine Löslichkeit von wenigstens 4 g Aktivsubstanz pro 100 g Lösung bei 20 °C verstanden. Erfindungsgemäß verwendbar sind beispielsweise Alaun (KAI(SO₄)₂. 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Aluminiumchlorohydrate werden beispielsweise pulverförmig als Micro Dry^{®} Ultrafine oder in aktivierter Form als Reach^{®} 501 oder Reach^{®} 103 von Reheis sowie in Form wäßriger Lösungen als Locron^{®} L von Clariant oder als Chlorhydrol^{®} von Reheis vertrieben. Unter der Bezeichnung Reach^{®} 301 wird ein Aluminiumsesquichlorohydrat von Reheis angeboten. Auch die Verwendung von Aluminium-Zirkonium-Tri- oder Tetrachlorohydrex-Glycin-Komplexen, die beispielsweise von Reheis unter der Bezeichnung Rezal^{®} 36G im Handel sind, ist erfindungsgemäß besonders vorteilhaft.

Der schweißhemmende Wirkstoff ist in den erfindungsgemäßen Zusammensetzungen in einer Menge von 0,01 - 40 Gew.-%, vorzugsweise 2 - 30 Gew.-% und insbesondere 5 - 25 Gew.-%, bezogen auf die Menge der Aktivsubstanz in der gesamten Zusammensetzung, enthalten.

In der erfindungsgemäßen Ausführungsform als Deodorant können Deodorant-Wirkstoffe enthalten sein. Erfindungsgemäß als Deodorant-Wirkstoffe geeignet sind Duftstoffe, antimikrobielle, antibakterielle oder keimhemmende Stoffe, enzymhemmende Stoffe, Antioxidantien und Geruchsadsorbentien.

Geeignete antimikrobielle, antibakterielle oder keimhemmende Stoffe sind insbesondere C₁-C₄-Alkanole, C₂-C₄-Alkandiole, Organohalogenverbindungen sowie -halogenide, quartäre Ammoniumverbindungen, eine Reihe von Pflanzenextrakten und Zinkverbindungen.

In einer weiteren bevorzugten Ausführungsform enthalten die erfindungsgemäßen Zusammensetzungen mindestens ein wasserlösliches Polyol, ausgewählt aus wasserlöslichen Diolen, Triolen und höherwertigen Alkoholen sowie Polyethylenglycolen. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.

Die erfindungsgemäßen Zusammensetzungen enthalten das wasserlösliche Polyol in Mengen von 1 - 50 Gew.-%, bevorzugt 1 - 15 Gew.-% und besonders bevorzugt 1 - 5 Gew.-%, jeweils bezogen auf die gesamte Zusammensetzung.

Die folgenden Beispiele sollen die vorliegende Erfindung verdeutlichen, ohne sie hierauf zu beschränken.

### Ausführungsbeispiele

### Beispiel 1: Beeinflussung des Wachstum von Staphylococcus warneri und Propionibacterium acnes durch Pflanzenextrakte

Aus flüssigen Vorkukulturen von S. *warneri* und *P. acnes* wurden Kulturen in LB-Medium mit einer Optischen Dichte OD (620nm) von 0,05 angeimpft. Parallel zu den Kontrollen (ohne Zusatz von Zellen bzw. Extrakt) wurden je 2 Kulturen mit 1 % Pflanzenextrakt versetzt und das Wachstum über 30 h anhand der Messung der OD dokumentiert. Nach 30 h wurde die Differenz der OD der Kulturen mit Extraktzusatz zu den entsprechenden Kontrollen (ohne Extraktzusatz, bereinigt um den Wert ohne Zellen) bestimmt. Ein Mischextrakt aus *Ribes nigrum* und *Pinus sylvestris* (Epica®, Rahn (Deutschland)) sowie Extrakte aus *Picea glauca, Paullinia cupana* (Guarana), *Panax ginseng* (Ginseng), *Lamium album* (White Nettle, Weisse Taubnessel) und *Ribes nigrum* (Blackcurrant, Schwarze Johannisbeere) hatten einen wachstumsfördernden Effekt auf *S*. *warneri* bei gleichzeitiger Hemmung von *P. acnes*. Die Extrakte wurden von der Firma Rahn (Deutschland) bezogen.

**Tabelle 1: Wachstumseffekte verschiedener Pflanzenextrakte auf S. warneri und P. acnes (OD-Differenzen zur Kontrolle nach 30 h)**

| | Guarana | Ribes nigrum/ Pinus sylvestris | Ginseng | White Nettle | Ribes nigrum | Picea glauca |
|---|---|---|---|---|---|---|
| S. warneri | 0,41 | 0,22 | 0,48 | 0,01 | 0,13 | 0,04 |
| P. acnes | -0,04 | -0,07 | -0,02 | -0,001 | -0,02 | -0,05 |

### Rezepturbeispiele

Die Angaben beziehen sich jeweils auf den Anteil in Gew.-%.

| **Bestandteil** | **Lamellare Creme, O/W-Emulsion [Gew.-%]** |
|---|---|
| Cetiol^{®} OE | 7,0 |
| Cetiol^{®} V | 7,0 |
| Lanette^{®} 22 | 7,0 |
| Lanette^{®} E | 0,18 |
| Baysilonöl M 350 | 0,5 |
| Vitamin E-acetat | 1,0 |
| Retinylpalmitat | 1,0 |
| D-Panthenol | 1,0 |
| Präbiotisches Pflanzenextrakt | 1,0 |
| Glycerol | 5,0 |
| Formalin-Lösung (37 %ig) | 0,08 |
| Wasser | ad 100 |

| **Bestandteil** | **Beispiel 3.1 O/W-PIT-Emulsion [Gew.-%]** | **Beispiel 3.2 W/O-Emulsion [Gew.-%]** |
|---|---|---|
| Cetiol^{®} OE | 7,5 | 7,0 |
| Cetiol^{®} V | 7,5 | 7,0 |
| Lanette^{®} O | 4,0 | - |
| Glycerylpalmitat | 2,2 | - |
| Eumulgin^{®} B 2 | 2,1 | - |
| Baysilonöl M 350 | 0,5 | - |
| Vitamin E-acetat | 1,0 | - |
| Retinylpalmitat | 1,0 | 1,0 |
| Biotin | 0,005 | - |
| Dihydro-3-hydroxy-4,4-dimethyl-2(3H)-furanon (Pantolacton) | 1,0 | 1,0 |
| Algenextrakt SPHM 3002 | - | 1,0 |
| Präbiotisches Pflanzenextrakt | 1,0 | 1,0 |
| Glycerol | 5,0 | 5,0 |
| MgSO₄·H₂O | - | 0,7 |
| Formalin-Lösung (37 %ig) | 0.08 | 0,08 |
| Wasser | ad 100 | ad 100 |

| **Bestandteil** | **Beispiel 3.3 Lipoprotein-Creme** | **Beispiel 3.4 Glycolipid-Creme** | **Beispiel 3.5** |
|---|---|---|---|
| Montanov^{®} 202 | - | - | 4,0 |
| Distelöl | 3,0 | - | - |
| Nachtkerzenöl | - | 3,0 | - |
| Myritol^{®} PC | 3,5 | 3,5 | - |
| Myritol^{®} 331 | - | - | 3,0 |
| Myritol^{®} 318 | - | - | 2,0 |
| Cetiol^{®} MM | - | 2,5 | - |
| Cetiol^{®} B | - | - | 7,0 |
| Cetiol^{®} SB 45 | - | - | 0,5 |
| Lanette^{®} 22 | 3,0 | - | - |
| Cutina^{®} GMS-V | 3,0 | 4,0 | 2,0 _ |
| Lanette^{®} O | 3,0 | 2,0 | 1,0 |
| Edenor^{®} IPS | 6,0 | 6,0 | - |
| Cosmacol^{®} PLG | - | 3,0 | - |
| Baysilonöl M350 | 1,0 | 1,0 | 0,5 |
| Eusolex^{®} 6300 | 0,6 | 0,6 | 3,0 |
| Parsol^{®} 1789 | 0,1 | 0,1 | 2,0 |
| Controx^{®} KS | 0,05 | 0,05 | 0,05 |
| pHB-Propylester | 0,2 | - | 0,2 |
| Präbiot. Pflanzenextrakt | 1,0 | 1,0 | 1,0 |
| Panthenol | 1,0 | 1,0 | 1,0 |
| Herbasol^{®} Destillat Malve | - | 1,0 | - |
| Herbasol^{®} Extrakt Rosmarin | - | - | 1,0 |
| Dry Flo^{®} Plus | - | 3,0 | - |
| Hexandiol-1,6 | - | 6,0 | - |
| Dipropylenglycol | - | 5,0 | - |
| Glycerol | 5,0 | - | - |
| DSC-H N | - | 5,0 | - |
| V- Protein flüssig | 9,0 | - | - |
| Tioveil^{®}-AQ-N | 2,0 | - | - |
| Citronensäure | 0,1 | - | - |
| Sepigel^{®} 305 | 3,0 | 0,4 | - |
| Methocel^{®} E 4M | - | - | 0,2 |
| Herbasol^{®} Destillat Grüner Tee | - | 1,0 | - |
| Wasser | ad 100 | ad 100 | ad 100 |

| **Bestandteil** | **Beispiel 3.6 Mildes Reinigungsgel** |
|---|---|
| Eumulgin^{®} HRE 40 | 0,6 |
| Eucarol^{®} AGE-ET | 2,0 |
| 1,2-Propylenglycol | 10,0 |
| Präbiotisches Pflanzenextrakt | 1,0 |
| Bisabolol | 0,1 |
| D-Panthenol | 0,5 |
| pHB- Propylester | 0,1 |
| pHB- Methylester | 0,2 |
| Carbopol^{®} ETD 2020 (0,5%ig) | 50,0 |
| Wasser | ad 100 |

| **Bestandteil** | **Beispiel 3.7 Matrixpflaster** | **Beispiel 3.8 Matrixpflaster** |
|---|---|---|
| DURO-TAK^{®} | 76 | 76 |
| Präbiotisches Pflanzenextrakt | 1,0 | 1,0 |
| Ultrasome™ | - | 0,1 |
| Panthenol | 2 | - |
| Dihydro-3-hydroxy-4,4-dimethyl-2(3H)-furanon (Pantolacton) | - | 2 |
| Herbasol^{®} Destillat Eibisch | 1 | - |
| Herbasol^{®} Destillat Grüner Tee | - | 1 |
| Aloe Vera Gel | 1 | 1 |
| Tioveil^{®}-AQ-N | 2 | - |
| Eusolex^{®} OCR | 1 | - |
| Propylenglycolmonooleat | 5 | 5 |
| Controx^{®} KS | 0,05 | 0,05 |
| Wasser | ad 100 | ad 100 |

| **Bestandteile des Wirkstoffreservoirs** | **Beispiel 3.9 Gel-Reservoirpflaster** | **Beispiel 3.10 Gel-Reservoirpflaster** |
|---|---|---|
| Präbiotisches Pflanzenextrakt | 1,0 | 1,0 |
| Ultrasome™ | - | 0,1 |
| Panthenol | 1,0 | - |
| Pantolacton | | 1,0 |
| Bisabolol | 1,0 | 1,0 |
| Herbasol^{®} Destillat Eibisch | - | 1,0 |
| Ethanol | 40 | 40 |
| Mowiol^{®} 18-88 | 8,0 | 8,0 |
| Luviskol^{®} K 80 | 5,0 | 5,0 |
| Controx^{®} KS | 0,05 | 0,05 |
| Brij^{®}-35 | 2,0 | 2,0 |
| Cremophor^{®} CO-40 | 0,5 | 0,5 |
| Glycerol | 5,0 | 5,0 |
| Wasser | ad 100 | ad 100 |

### 4. Öl-in-Wasser-Emulsionen

### 4.1. Beispielserie:

| | **1** | **2** | **3** |
|---|---|---|---|
| Carthamus Tinctorius | 3,00 | 3,00 | 3,00 |
| Caprylic/Capric Triglyceride | 5,00 | 5,00 | 5,00 |
| Cocoglycerides | 2,00 | 2,00 | 2,00 |
| Behenyl Alcohol | 1,00 | 1,00 | 1,00 |
| Glyceryl Stearate | 2,00 | 2,00 | 2,00 |
| Cetearyl Alcohol | 1,00 | 1,00 | 1,00 |
| Isopropylstearate | 4,00 | 4,00 | 4,00 |
| Shea Butter | 2,00 | 2,00 | 2,00 |
| Dimethicone | 1,00 | 1,00 | 1,00 |
| Hydrogenated Palm Glycerides Citrate | 0,05 | 0,05 | 0,05 |
| Propylparaben | 0,20 | 0,20 | 0,20 |
| Cyclopentasiloxane/Dimethiconol | 1,00 | 1,00 | 1,00 |

| Aluminium Starch Octenylsuccinate | 1,00 | 1,00 | 1,00 |
|---|---|---|---|
| TiO2 | 0,50 | 0,50 | 0,50 |
| Hexandiol | 6,00 | 3,00 | |
| Propylenglycol | 5,00 | 5,00 | 5,00 |
| Glycerine | 5,00 | 3,00 | 3,00 |
| Methylparaben | 0,20 | 0,20 | 0,20 |
| Sodium Carbomer | 0,40 | 0,40 | 0,40 |
| Dimethylsilanol Hyaluronate | 5,00 | 5,00 | 5,00 |
| Algea Extract | 1,00 | | |
| Präbiotisches Pflanzenextrakt | 0,5 | 0,8 | 1,2 |
| Perfume | 0,10 | 0,10 | 0,10 |
| Aqua | ad. 100 | ad. 100 | ad. 100 |

### 4.2. Beispielserie:

| | **1** | **2** | **3** |
|---|---|---|---|
| Cetearyl Isononanoate | 4,00 | 4,00 | 4,00 |
| Mineral oil | 6,00 | 6,00 | 6,00 |
| Glyceryl Stearate / Cetearyl Alcohol / Cetyl Palmitate / Cocoglycerides | 2,00 | 2,00 | 2,00 |
| Palmitic Acid / Stearic Acid | 1,50 | 1,50 | 1,50 |
| Ceteareth-30 | 1,00 | 1,00 | 1,00 |
| Dimethicone | 1,00 | 1,00 | 1,00 |
| Tocoperyl Acetate | 0,50 | 0,50 | 0,50 |
| Propylparaben | 0,30 | 0,30 | 0,30 |
| Sweet Almond Oil | 2,00 | 2,00 | 2,00 |
| Acrylates/C10-30 Alkyl Acrylate Crosspolymer | 0,27 | 0,27 | 0,27 |
| Glycerine | 5,00 | 3,00 | 3,00 |
| Lactic Acid | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | | |
| Laminaria Digitata Extract | 1,00 | | |
| Sodium Chloride | 0,05 | 0,05 | 0,05 |
| Polyacrylamide / C13-14 Isoparaffin/Laureth-7 | 0,50 | 0,50 | 0,50 |
| Silk Protein | 0,25 | 0,25 | 0,25 |
| Xanthan Gum | 0,20 | 0,20 | 0,20 |
| Präbiotisches Pflanzenextrakt | 0,6 | 0,8 | 1,0 |
| Perfume | 0,30 | 0,30 | 0,30 |
| Aqua | ad. 100 | ad. 100 | ad.100 |

### 4.3. Beispielserie:

| | **1** | **2** | **3** |
|---|---|---|---|
| Hydrogenated Lecithin | 0,50 | 0,50 | 0,50 |
| Isopropylstearate | 4,00 | 4,00 | 4,00 |
| Dibutyl Adipate | 2,00 | 2,00 | 2,00 |
| Tocopheryl Acetate | 0,50 | 0,50 | 0,50 |
| Glyceryl Stearate | 1,00 | 1,00 | 1,00 |
| Behenyl Alcohol | 2,00 | 2,00 | 2,00 |
| Dimethicone | 0,50 | 0,50 | 0,50 |
| Propylparaben | 0,20 | 0,20 | 0,20 |
| Cyclomethicone/ Dimethicone Crosspolymer | 1,00 | 1,00 | 1,00 |
| Glycerine | 4,50 | 3,00 | 3,00 |
| Hexandiol | 6,00 | 3,00 | |
| Methylparaben | 0,20 | 0,20 | 0,20 |
| Sodium Carbomer | 0,30 | 0,30 | 0,30 |
| Dimethyl Silanol Hyaluronate | 5,00 | 5,00 | 5,00 |
| Algae Extract | 1,00 | | |
| Plancton Extract | 0,20 | 0,20 | 0,20 |
| Dimethylmethoxy Chromanol | 0,01 | 0,01 | 0,01 |
| Propylenglycol | 5,00 | 5,00 | 5,00 |
| Palmitoyl Pentapeptide-3 | 3,00 | | |
| Palmitoyl Oligopeptide | | 2,00 | |
| Palmitoyl Tetrapeptide-1 | | 1,00 | |
| Hydroxyethyl Acrylate / Sodium Acryloyldimethyl Taurate Copolymer / Squalane / Polysorbate 60 | 1,50 | 1,50 | 1,50 |
| Ti02 | 0,50 | 0,50 | |
| Präbiotisches Pflanzenextrakt | 0,5 | 0,9 | 1,3 |
| Perfume | 0,35 | 0,35 | 0,35 |
| Aqua | ad. 100 | ad. 100 | ad.100 |

### 4.4. Beispielserie:

| | **1** | **2** | **3** | **4** | **5** |
|---|---|---|---|---|---|
| Cetearyl Alkohol / Cetearyl Glucoside | 5,00 | 5,00 | 5,00 | 5,00 | 5,00 |
| Caprylic/Capric Triglyceride | 5,00 | 5,00 | 5,00 | 5,00 | 5,00 |
| Shea Butter | 0,50 | 0,50 | 0,50 | 0,50 | 0,50 |
| Cocoglycerides | 2,00 | 2,00 | 2,00 | 2,00 | 2,00 |
| Cetearyl Alkohol | 1,00 | 1,00 | 1,00 | 1,00 | 1,00 |
| Glyceryl Stearate | 2,00 | 2,00 | 2,00 | 2,00 | 2,00 |
| Dimethicone | 0,50 | 0,50 | 0,50 | 0,50 | 0,50 |
| Tocoperyl acetate | 0,50 | 0,50 | 0,50 | 0,50 | 0,50 |
| Hydrogenated Palm Glycerides Citrate | 0,25 | 0,25 | 0,25 | 0,25 | 0,25 |
| Polysilicone-15 | 4,00 | 4,00 | 4,00 | | |
| Phenylbenzimidazole Sulfonic Acid | | 2,00 | 2,00 | 2,00 | |
| Disodium Phenyl Dibenzimidazole Tetrasulfonate | | | | 1,00 | |
| Octocrylene | | | | 5,00 | |
| 4-Methoxybenzylidene Camphor | 2,00 | | | | |
| Buthyl-Methoxydibenzoylmethane | 1,00 | 1,80 | 1,80 | | |
| Propylparaben | 0,20 | 0,20 | 0,20 | 0,20 | 0,20 |
| Sodium Carbomer | 0,50 | 0,50 | 0,50 | 0,50 | 0,50 |
| Hexandiol | 6,00 | 6,00 | 3,00 | 6,00 | |
| Talc | 0,50 | 0,50 | 0,50 | 0,50 | 0,50 |
| Methylparaben | 0,20 | 0,20 | 0,20 | 0,20 | 0,20 |
| Glycerine | 4,50 | 4,50 | 3,00 | 4,50 | 3,00 |
| Aluminium Starch Octenylsuccinate | 1,00 | 1,00 | 1,00 | 1,00 | 1,00 |
| Hydrogenated Lecithin | 1,00 | 1,00 | 1,00 | 1,00 | 1,00 |
| Fagus Silvatica Extract | 2,00 | 2,00 | 2,00 | 2,00 | 2,00 |
| Hydrolyzed Soy Protein | 2,00 | 2,00 | 2,00 | 2,00 | 2,00 |
| Dimethylsilanol Hyaluronate | 2,00 | 2,00 | 2,00 | 2,00 | 2,00 |
| Trisodium NTA | 0,10 | 0,10 | 0,10 | 0,10 | 0,10 |
| Phenoxyethanol | 0,40 | 0,40 | 0,40 | 0,40 | 0,40 |
| Hydrolyzed Wheat Protein | 3,00 | 3,00 | 3,00 | 3,00 | 3,00 |
| Präbiotisches Pflanzenextrakt | 0,8 | 1,2 | 1,0 | 1,0 | 1,2 |
| Perfume | 0,40 | 0,40 | 0,40 | 0,40 | 0,40 |
| Aqua | ad. 100 | ad. 100 | ad. 100 | ad. 100 | ad. 100 |

### 4.5. Beispielserie:

| | **1** | **2** | **3** |
|---|---|---|---|
| C14-22 Alcohols/C12-20 Alkyl Glucoside | 3,00 | 3,00 | 3,00 |
| Dibutyl Adipate | 6,00 | 6,00 | 6,00 |
| Caprylic/Capric Triglyceride | 3,00 | 3,00 | 3,00 |
| Cetearyl Alkohol | 1,00 | 1,00 | 1,00 |
| Glyceryl Stearate | 0,50 | 0,50 | 0,50 |
| Dimethicone | 0,50 | 0,50 | 0,50 |
| Propylparaben | 0,20 | 0,20 | 0,20 |
| Cyclopentasiloxane | 1,50 | 1,50 | 1,50 |
| Glycerine | 5,00 | 5,00 | 5,00 |
| Sorbitol | 3,00 | 3,00 | 3,00 |
| Methylparaben | 0,20 | 0,20 | 0,20 |
| Aluminium Starch Octenylsuccinate | 0,50 | 0,50 | 0,50 |
| Sodium Carbomer | 0,10 | 0,10 | 0,10 |
| Dimethylsilanol Hyaluronate | 2,00 | 2,00 | 2,00 |
| Laminata Digitata Extract | 0,50 | 0,50 | 0,50 |
| Hydroxyethyl Acrylate / Sodium Acryloyldimethyl Taurate Copolymer / Squalane / Polysorbate 60 | 1,00 | 1,00 | 1,00 |
| Präbiotisches Pflanzenextrakt | 0,4 | 0,8 | 1,2 |
| Perfume | 0,20 | 0,20 | 0,20 |
| Aqua | ad. 100 | ad. 100 | ad. 100 |

### 5. Wasser-in-Öl-Emulsion

| | **1** | **2** | **3** |
|---|---|---|---|
| Polyglyceryl-3 Diisostearate | 3,0 | 3,0 | 3,0 |
| PEG-45/Dodecyl Glycol Copolymer | 0,5 | 0,5 | 0,5 |
| Microcrystalline Wax | 3,0 | 3,0 | 3,0 |
| Bis-Diglyceryl Polyacyladipate-2 | 1,0 | 1,0 | 1,0 |
| Paraffinöl | 8,0 | 8,0 | 8,0 |
| Vaseline | 2,0 | 2,0 | 2,0 |
| Vitamin-E-acetat | 2,0 | 2,0 | 2,0 |
| Methylparaben | 0,3 | 0,3 | 0,3 |
| Propylparaben | 0,3 | 0,3 | 0,3 |
| Isopropylisostearate | 8,0 | 8,0 | 8,0 |
| Glycerin | 5,0 | 3,0 | 3,0 |
| Mg-Sulfat | 0,5 | 0,5 | 0,5 |
| Pflanzenextrakt | 0,8 | 1,2 | 1,5 |
| Milchsäure 80%ig | 0,560 | 0,560 | 0,560 |
| Algae Extract | 1,0 | | |
| Panthenol | 0,5 | 1,0 | 0,5 |
| Calendula Officinalis Flower Extract | 0,3 | | |
| Propylene Glycol | 0,5 | | |
| Parfum | 0,2 | 0,2 | 0,2 |
| Aqua | ad.100 | ad.100 | ad.100 |

### 6. Reinigungszubereitungen

### 6.1. Beispielserie:

| | **1** | **2** | **3** |
|---|---|---|---|
| Dipropylenglycol | 10,00 | 10,00 | 10,00 |
| Chlorhexidindigluconate | 1,00 | 1,00 | 1,00 |
| Poloxamer-184 | 3,00 | 3,00 | 3,00 |
| Panthenol | 0,50 | 0,50 | 0,50 |
| Chitosan Glycolate | 3,00 | 3,00 | 3,00 |
| PEG-40 Hydrogenated Castor Oil / Trideceth 9 / Propylen Glycol | 0,50 | 0,50 | 0,50 |
| Chlorella Vulgaris Extract | 0,50 | 0,50 | 0,50 |
| Präbiotisches Pflanzenextrakt | 0,4 | 0,8 | 1,2 |
| Perfume | '0,20 | 0,20 | 0,20 |
| Aqua | ad. 100 | ad. 100 | ad. 100 |

### 6.2. Beispielserie:

| | **1** | **2** | **3** |
|---|---|---|---|
| Carbomer | 1,40 | 1,40 | 1,40 |
| Sorbitol | 2,10 | 2,10 | 2,10 |
| Sodium Benzoate | 0,40 | 0,40 | 0,40 |
| Lauryl Glucoside | 7,50 | 7,50 | 7,50 |
| Cocoamidopropyl beatine | 3,40 | 3,40 | 3,40 |
| Disodium Laureth Sulfosuccinate | 5,00 | 5,00 | 5,00 |
| PEG-7 Glyceryl Cocoate | 0,50 | 0,50 | 0,50 |
| Coco-Glucoside / Glyceryl Oleate | 5,00 | 5,00 | 5,00 |
| Hydrogenated Palm Glycerides Citrate | 0,05 | 0,05 | 0,05 |
| Sodium PCA | 1,60 | 1,60 | |
| Pantolactone | 1,00 | 1,00 | |
| Tetrasodium EDTA | 0,25 | 0,25 | 0,25 |
| Sodium Lactate | 1,80 | | |
| Panthenol | 0,50 | 0,50 | 0,50 |
| Präbiotisches Pflanzenextrakt | 0,8 | 1,2 | 1,6 |
| Perfume | 0,40 | 0,40 | 0,40 |
| Aqua | ad. 100 | ad. 100 | ad. 100 |

### 7. Wasser-in-Silicon-Emulsion

| | **1** | **2** | **3** |
|---|---|---|---|
| Dimethicone | 2,50 | 2,50 | 2,50 |
| Cyclomethicone | 25,00 | 25,00 | 25,00 |
| Cetyl Dimethicone Copolyol | 2,00 | 2,00 | 2,00 |
| Sodium Chloride | 2,00 | 2,00 | 2,00 |
| Perfume | 0,30 | 0,30 | 0,30 |
| Chlorhexidindigluconate | 0,20 | 0,20 | 0,20 |
| Präbiotisches Pflanzenextrakt | 0,4 | 0,8 | 1,2 |
| Aqua | ad. 100 | ad. 100 | ad. 100 |

### Verwendete Rohstoffe:

| **Name** | **INCI** |
|---|---|
| Algenextrakt SPHM 3002 | Aqua, Algae (Linne) |
| Aloe Vera Gel (Provital SA): 0,85 - 1,55 Gew.-% Aktivsubstanz in Propylenglykol / Wasser | Aloe Barbadensis (Linne) |
| Baysilonöl M 350 | Polydimethylsiloxan/Dimethicone |
| Brij^{®}-35 | Laureth-23 |
| Carbopol^{®} ETD 2020 (0,5%ig) | Acrylates/C10-30 Alkyl Acrylate Crosspolymer |
| Eumulgin^{®} B 2 | Ceteareth-20 |
| Cetiol^{®} B | Dibutyl Adipate |
| Cetiol^{®} MM | Myristyl Myristate |
| Cetiol^{®} SB 45 | Butyrospermum Parkii (Linne) |
| Controx^{®} KS: | Tocopherol, Hydrogenated Palm Glycerides Citrate |
| Cosmacol^{®} PLG | Tri-C12-13 Alkyl Citrate |
| Cremophor^{®} CO-40 | PEG-40 Hydrogenated Castor Oil |
| Cutina^{®} GMS (C16-18-Fettsäure-mono-di-glycerid | Glyceryl Stearate |
| Cetiol^{®} V | Decyl Oleate |
| Cetiol^{®} OE | Dicaprylylether |
| Dry Flo^{®} Plus | Aluminium Starch Octenylsuccinate |
| DSC-H N (ex Exsymol) | Dimethylsilanol Hyaluronate |
| DURO-TAK^{®} (National Starch and Chemical): ca. 50 % Acrylat-Copolymer in Benzin/Ethylacetat/Methanol/Ethanol | Polyacrylate Copolymer |
| Eucarol^{®} AGE-ET UP (30% Aktivsubstanz in Wasser) | Sodium Cocopolyglucose Tartrate |
| Eumulgin^{®} HRE 40 | PEG-40 Hydrogenated Castor Oil |
| Eusolex^{®} 6300 | 4-Methytbenzylidene Camphor |
| Eusolex^{®} OCR: | Octocrylene |
| Herbasol^{®} Destillat Eibisch (Cosmetochem) | Water, Alcohol denat., Althea officinalis |
| Herbasol^{®} Destillat Grüner Tee | Water, Camellia sinensis extract |
| Herbasol^{®} Destillat Malve (Cosmetochem) | Aqua, SD Alcohol 39-C, Malva Sylvestris (Linne) |
| Herbasol^{®} Extrakt Rosmarin | Water, Propylene Glycol, Rosmarinus officinalis |
| Edenor^{®} IPS | Isopropyl Stearate |
| Lanette^{®} E | Sodium Cetearyl Sulfate |
| Lanette^{®} O | Cetearyl Alcohol |
| Lanette^{®} 22 | Behenyl Alcohol |
| Lifidrem^{®} PPST-GHK-4 (Coletica): Erbsenprotein-Extrakt/C₁₆₋₁₈-Fettsäure-Kondensat | Pea Extract (Pisum Sativum (Linne)), Sodium Stearate, Sodium Chloride |
| Methocel^{®} E 4M | Hydroxypropyl Methylcellulose |
| Montanov^{®} 202 | Arachidyl Alcohol, Behenyl Alcohol, Arachidyl Glucoside |
| Myritol^{®} 318 | Caprylic/Capric Triglyceride |
| Myritol^{®} 331 | Cocoglycerides |
| Myritol^{®} PC | Propylene Glycol Dicaprylate/Dicaprate |
| Nachtkerzenöl | Evening Primrose Oil Oenothera Biennis (Linne) |
| Parsol^{®} 1789 | Butyl Methoxydibenzoylmethane |
| pHB-Propylester | Propylparaben |
| Photosome™ | Plankton Extract and Lecithin |
| Mowiol^{®} 18-88 | Polyvinylalkohol, teilverseift |
| Luviskol^{®} K 80 | Polyvinylpyrrolidon |
| Sepigel^{®} 305 | Polyacrylamide, C13-14 Isoparaffin, Laureth-7 |
| Tioveil^{®}-AQ-N (Uniqema):Titandioxid-Dispersion | CI 77891 (Titanium Dioxide), Alumina, Silica, Sodium Polyacrylate |
| Ultrasome™ | Micrococcus lysate |
| V-Protein flüssig COS 152/22 A (Cosmetochem) | Aqua, Propylene Glycol, Hydrolyzed Pea Protein (Pisum Sativum) |
| Vitamin E-acetat | Tocopheryl Acetate |

## Patentansprüche

1. Kosmetische oder pharmazeutische Zusammensetzung, enthaltend eine Extraktmischung aus *Ribes nigrum* und *Pinus sylvestris.*

2. Kosmetische oder pharmazeutische Zusammensetzung nach Anspruch 1, **dadurch gekennzeichnet, dass** es sich um ein topisches Hautbehandlungsmittel handelt.

3. Kosmetische oder pharmazeutische Zusammensetzung nach einem der Ansprüche 1 oder 2, **dadurch gekennzeichnet, dass** der Pflanzenextrakt in einer Menge von 0,01 bis 20 Gew.-%, bezogen auf das Gesamtgewicht der Zusammensetzung, enthalten ist.

4. Kosmetische oder pharmazeutische Zusammensetzung nach einem der Ansprüche 1, 2 oder 3, **dadurch gekennzeichnet, dass** sie mindestens eine weitere Substanz enthält, ausgewählt aus mindestens einer der folgenden Gruppen:
- organische, mineralische und modifizierte mineralische Lichtschutzfilter,
- Vitamine, Provitamine oder Vitaminvorstufen der Vitamin B-Gruppe oder deren Derivate sowie Derivate von 2-Furanon, Panthenol, Pantolacton, Nicotinsäureamid und Biotin,
- weitere Pflanzenextrakte,
- MMP-1-inhibierende Substanzen,
- Ester von Retinol (Vitamin A₁) mit einer C₂₋₁₈-Carbonsäure.

5. Kosmetische oder pharmazeutische Zusammensetzung nach einem der Ansprüche 1, 2, 3 oder 4, **dadurch gekennzeichnet, dass** sie mindestens eine weitere Substanz enthält, ausgewählt aus mindestens einer der folgenden Gruppen:
- oberflächenaktive Substanzen als Emulgator oder Dispergiermittel,
- Aminosäuren und deren Zinksalze und deren Säureadditionssalze,
- filmbildende und/oder emulsionsstabilisierende und/oder verdickende und/oder adhäsive Polymere,
- Fettstoffe, Tenside, Anti-Transpirantien und Polyole,
- organische, mineralische und modifizierte mineralische Lichtschutzfilter,
- Proteinhydrolysate und deren Derivaten,
- Mono-, Oligo- und Polysaccharide sowie deren Derivate,
- α-Hydroxycarbonsäuren und α-Ketocarbonsäuren sowie deren Ester-, Lacton- oder Salzformen.

6. Kosmetische oder pharmazeutische Zusammensetzung nach einem der Ansprüche 1, 2, 3, 4 oder 5, **dadurch gekennzeichnet, dass** diese in einer Seife, einer Lotion, einer Emulsion, einem Spray, einer Creme, einem Shampoo oder einem Pflaster enthalten ist.

7. Verwendung eines Extrakts aus *Ribes nigrum* zur Herstellung topischer pharmazeutischer oder kosmetischer Zusammensetzungen zur Förderung des Wachstums Koagulase-negativer Staphylokokken, ausgewählt aus *S. epidermidis* und *S. wameri,* und zur gleichzeitigen Hemmung des Wachstums von *Propionibacterium acnes* auf der Haut.

8. Verwendung nach Anspruch 7, **dadurch gekennzeichnet, dass** ein Extrakt aus *Pinus sylvestris* enthalten ist.

9. Verwendung nach Anspruch 7 oder 8 zur Behandlung unreiner oder fettiger Haut und zur Behandlung von Akne.

10. Verwendung nach einem der Ansprüche 7 bis 9, **dadurch gekennzeichnet, dass** die Hautbehandlung präventiv erfolgt.

11. Verwendung nach einem der Ansprüche 7 bis 10, **dadurch gekennzeichnet, dass** der Pflanzenextrakt in einer Menge von 0,01 bis 20 Gew.-%, bezogen auf das Gesamtgewicht der Zusammensetzung, enthalten ist.

## Claims

1. Cosmetic or pharmaceutical composition, comprising an extract mixture of *Ribes nigrum* and *Pinus sylvestris.*

2. Cosmetic or pharmaceutical composition according to claim 1, **characterized in that** said composition is a topical skin treatment agent.

3. Cosmetic or pharmaceutical composition according to one of claims 1 or 2, **characterized in that** the plant extract is comprised in an amount of 0.01 to 20 wt.-%, based on the total weight of the composition.

4. Cosmetic or pharmaceutical composition according to one of claims 1, 2 or 3, wherein said composition comprises at least one additional substance, selected from at least one of the following groups:
- organic, mineral and modified mineral light protecting filters,
- vitamins, provitamins or vitamin precursors of the vitamin B group or their derivatives as well as the derivatives of 2-furanone, panthenol, pantolactone, nicotinamide and biotin,
- additional plant extracts,
- MMP-1-inhibiting substances,
- esters of retinol (vitamin A₁) with a C₂₋₁₈ carboxylic acid.

5. Cosmetic or pharmaceutical composition according to one of claims 1, 2, 3 or 4, wherein said composition comprises at least one additional substance, selected from at least one of the following groups:
- surface active substances as the emulsifier or dispersion agent,
- amino acids and their zinc salts and their acid addition salts,
- film-forming and/or emulsion-stabilizing and/or thickening and/or adhesive polymers,
- fats, surfactants, antiperspirants and polyols,
- organic, mineral and modified mineral light protecting filters,
- protein hydrolyzates and their derivatives,
- mono-, oligo- and polysaccharides as well as their derivatives,
- α-hydroxycarboxylic acids and α-ketocarboxylic acids as well as their esters, lactones or salts.

6. Cosmetic or pharmaceutical composition according to one of claims 1, 2, 3, 4 or 5, wherein said composition is comprised in a soap, a lotion, an emulsion, a spray, a cream, a shampoo or a plaster.

7. Use of an extract of *Ribes nigrum* for manufacturing topical pharmaceutical or cosmetic compositions for promoting the growth of coagulase-negative staphylococci, selected from *S. epidermidis* and *S. warneri,* and for concomitant inhibition of the growth of *Propionibacterium acnes* on the skin.

8. Use according to claim 7, **characterized in that** an extract of *Pinus sylvestris* is comprised.

9. Use according to claim 7 or 8 for treating bad or greasy skin and for treating acne.

10. Use according to one of claims 7 to 9, **characterized in that** the skin treatment is carried out preventatively.

11. Use according to one of claims 7 to 9, **characterized in that** the plant extract is comprised in an amount of 0.01 to 20 wt.-%, based on the total weight of the composition.

## Revendications

1. Composition cosmétique ou pharmaceutique, contenant un mélange d'extraits de *Ribes nigrum* et de *Pinus sylvestris.*

2. Composition cosmétique ou pharmaceutique selon la revendication 1, **caractérisée en ce qu'**il s'agit d'un agent de traitement cutané topique.

3. Composition cosmétique ou pharmaceutique selon l'une quelconque des revendications 1 ou 2, **caractérisée en ce que** l'extrait de plante est contenu en une quantité de 0,01 à 20 % en poids, rapportés au poids total de la composition.

4. Composition cosmétique ou pharmaceutique selon l'une quelconque des revendications 1, 2 ou 3, **caractérisée en ce qu'**elle contient au moins une substance supplémentaire choisie parmi au moins un des groupes suivants :
- des filtres organiques, minéraux et minéraux modifiés de protection contre la lumière ;
- des vitamines, des provitamines ou des précurseurs de vitamines du groupe des vitamines B, ou leurs dérivés, ainsi que des dérivés de la 2-furanone, du panthénol, de la pantolactone, de l'amide de l'acide nicotinique et de la biotine ;
- d'autres extraits de plantes ;
- des substances inhibitrices de MMP-1 ;
- des esters du rétinol (vitamine A₁) avec un acide carboxylique en C₂-C₁₈.

5. Composition cosmétique ou pharmaceutique selon l'une quelconque des revendications 1, 2, 3 ou 4, **caractérisée en ce qu'**elle contient au moins une substance supplémentaire choisie parmi au moins un des groupes suivants :
- des substances tensioactives à titre d'émulsifiant ou d'agent de mise en dispersion ;
- des acides aminés et leurs sels de zinc ainsi que leurs sels d'addition d'acides ;
- des polymères filmogènes et/ou de stabilisation de l'émulsion et/ou épaississants et/ou adhésifs ;
- des substances grasses, des agents tensioactifs, des antitranspirants et des polyols ;
- des filtres organiques, minéraux et minéraux modifiés de protection contre la lumière ;
- des hydrolysats de protéines et leurs dérivés ;
- des monosaccharides, des oligosaccharides et des polysaccharides ainsi que leurs dérivés ;
- des acides α-hydroxycarboxyliques et des acides α-cétocarboxyliques, ainsi que leurs formes d'esters, de lactones ou de sels.

6. Composition cosmétique ou pharmaceutique selon l'une quelconque des revendications 1, 2, 3, 4 ou 5, **caractérisée en ce qu'**elle est contenue dans un savon, dans une lotion, dans une émulsion, dans un spray, dans une crème, dans un shampooing ou dans un emplâtre.

7. Utilisation d'un extrait de *Ribes nigrum* pour la préparation de compositions pharmaceutiques ou cosmétiques topique destinée à favoriser la croissance des staphylocoques à coagulase négative, choisis parmi *S. epidermidis* et *S. wameri,* et à inhiber de manière simultanée la croissance de *Propionibacterium acnes* sur la peau.

8. Utilisation selon la revendication 7, **caractérisée en ce que** la composition contient un extrait de *Pinus sylvestris.*

9. Utilisation selon la revendication 7 ou 8, pour le traitement d'une peau impure ou grasse et pour le traitement de l'acné.

10. Utilisation selon l'une quelconque des revendications 7 à 9, **caractérisée en ce que** le traitement cutané a lieu à titre préventif.

11. Utilisation selon l'une quelconque des revendications 7 à 10, **caractérisée en ce que** l'extrait de plante est contenu en une quantité de 0,01 à 20 % en poids, rapportés au poids total de la composition.
